# EUROPEAN PATENT SPECIFICATION

(11) **EP 2 384 689 B1**
(45) Date of publication and mention of the grant of the patent: **07.12.2016**
(21) Application number: 10824738.8
(22) Date of filing: 14.09.2010
(51) Int. Cl.: A61B 1/04, A61B 1/00

(54) **FLUORESCENCE OBSERVATION DEVICE**
FLUORESZENZBEOBACHTUNGSGERÄT
DISPOSITIF POUR OBSERVER DES FLUORESCENCES

(30) Priority: 20.10.2009 JP 2009241286
(43) Date of publication of application: 09.11.2011
(73) Proprietor: Olympus Corporation, Tokyo 192-8507 (JP)
(72) Inventor: KUBO, Kei, Hachioji-shi, Tokyo 192-8507 (JP); DOGUCHI, Nobuyuki, Hachioji-shi, Tokyo 192-8507 (JP); IMAIZUMI, Katsuichi, Hachioji-shi, Tokyo 192-8507 (JP); TAKEI, Shunji, Hachioji-shi, Tokyo 192-8507 (JP)
(74) Representative: Gunzelmann, Rainer
(86) International application number: PCT/JP2010/065828
(87) International publication number: WO 2011/048887

(56) References cited:
- EP-A1- 2 098 156
- JP-A- 6 054 792
- JP-A- 2005 329 115
- JP-A- 2006 181 387
- JP-A- 2007 020 775
- JP-A- 2007 075 198
- JP-A- 2007 202 621
- US-A1- 2004 064 016
- US-A1- 2006 020 169

## Description

### Technical Field

The present invention relates to a fluorescence observation apparatus, and more particularly, to a fluorescence observation apparatus capable of observing fluorescent light beams emitted from a plurality of fluorescent substances. Background Art

Cancer diagnosis techniques using molecular targeting agents have come to be a focus of attention in recent years. To be more specific, for example, a technique of scattering or injecting into a target region of a living body a fluorescence probe (fluorescence agent) targeting biological proteins that are expressed specifically in cancer cells and then identifying the presence/absence of cancer based on fluorescent light emitted from the target region has been under study in recent years. Such a technique is useful for early detection of cancer in the field of the digestive tract.

Furthermore, as an application of the aforementioned technique, a technique is being proposed which is designed to scatter or inject a plurality of types of fluorescence probes of different fluorescent light wavelengths into a target region of a living body and observe an expression state of a plurality of types of biological proteins corresponding to the plurality of types of fluorescence probes in a composite manner based on a plurality of fluorescent light beams emitted from the target region. Such a technique is considered useful for estimation of stages of cancer, prediction of risk of cancer invasion and prediction of risk of cancer metastasis or the like.

For example, Japanese Patent Application Laid-Open Publication No. 2008-161550 discloses an endoscope system that makes observations by scattering or injecting a plurality of types of fluorescence probes into a target region of a living body, configured to be able to acquire a fluorescence image (image of a fluorescent light distribution) for each fluorescence probe by carrying out calculation processing based on a relationship between an intensity of fluorescent light and concentration of the fluorescence probe obtained during the observations.

Incidentally, in a fluorescence observation using fluorescence probes, even in a case where the same target region is observed, for example, brightness of fluorescent light beams sometimes differs depending on types of the fluorescence probes to be used. In addition, in a fluorescence observation using fluorescence probes, diagnosis is performed on a target region while viewing an observation image in which a fluorescence image corresponding to fluorescent light beams emitted from the fluorescence probes made to act on a target region and a reflected light image (reference light image) corresponding to the reflected light (reference light) reflected from the target region are superimposed on each other, for example.

Therefore, when a plurality of fluorescence probes are made to act simultaneously on the same target region, brightness differs among a plurality of fluorescence images acquired corresponding to a plurality of fluorescent light beams emitted from the plurality of fluorescence probes, which would cause a possibility that an observation image is outputted in which a fluorescence image which is too bright relative to brightness of a reflected light image and a fluorescence image which is too dark relative to brightness of the reflected image are superimposed on each other. In addition, in diagnosis while viewing such an observation image, there is a problem that both a part which is too bright relative to brightness of the reflected light image and a part which is too dark relative to brightness of the reflected light image are displayed in brightness which is not suitable for diagnosis.

On the other hand, the Japanese Patent Application Laid-Open Publication No. 2008-161550 is silent as to means for solving the above-described problem. Therefore, the technology disclosed in the Japanese Patent Application Laid-Open Publication No. 2008-161550 has such a problem that an image which will lead a decrease in diagnosis performance on a region to be observed is displayed in the observation image in which a plurality of fluorescence images and the reflected light image are superimposed on each other.

The present invention is defined by the appended claims, and an object of the present invention is to provide a fluorescence observation apparatus capable of improving diagnosis performance on a region to be observed by appropriately adjusting brightness difference among a plurality of fluorescence images acquired corresponding to a plurality of fluorescent light beams emitted from a plurality of fluorescence probes.

US 2006/020169 A1 discloses to uniformly mark a particular region of an image obtained from a single fluorescent light image which deviates in contrast from a predetermined threshold value with a particular predetermined color in order to make this region better visible to a surgeon. US 2006/020169 A1 does not teach to take into account different images obtained from corresponding fluorescent light beams, but only teaches to perform an adjustment so as to minimize a brightness difference between a white light image and a single florescent image that are adjacently displayed.

### Disclosure of Invention

### Means for Solving the Problem

A fluorescence observation apparatus according to the present invention includes a light source section that can emit a plurality of excitation light beams for exciting a plurality of fluorescent substances and a reference light, an image pickup section that picks up images of a plurality of fluorescent light beams emitted by emitting the plurality of excitation light beams to the plurality of fluorescent substances and reflected light of the reference light, an image generation section that generates image signals corresponding to the plurality of fluorescent light beams and the reflected light of the reference light whose images are picked up by the image pickup section, a brightness detection section that detects brightness of a plurality of image signals corresponding to the plurality of fluorescent light beams and brightness of an image signal corresponding to the reflected light of the reference light, and a brightness adjusting section that adjusts brightness of at least one image signal out of the plurality of image signals corresponding to the plurality of fluorescent light beams using brightness of the image signal corresponding to the reflected light of the reference light as a reference based on a detection result by the brightness detection section.

### Brief Description of the Drawings

Fig. 1 is a diagram illustrating a configuration of main parts of an endoscope system according to an embodiment of the present invention;
Fig. 2 is a diagram illustrating a filter switchover mechanism of an image pickup actuator when an optical filter is inserted in an optical path;
Fig. 3 is a diagram illustrating a state of a magnet displacement apparatus while current is being applied when the filter switchover mechanism is set in the state in Fig. 2;
Fig. 4 is a diagram illustrating the filter switchover mechanism of the image pickup actuator when the optical filter is retracted from the optical path;
Fig. 5 is a diagram illustrating a state of the magnet displacement apparatus while no current is being applied when the filter switchover mechanism is set in the state in Fig. 4;
Fig. 6 is a diagram illustrating characteristics of the optical filter provided in the image pickup actuator;
Fig. 7 is a diagram illustrating characteristics of an optical filter different from that in Fig. 6 provided in the image pickup actuator;
Fig. 8 is a diagram illustrating an example of configuration of a changeover filter provided in a light source apparatus;
Fig. 9 is a diagram illustrating characteristics of a normal light filter provided in the changeover filter;
Fig. 10 is a diagram illustrating characteristics of a first excitation light filter provided in the changeover filter;
Fig. 11 is a diagram illustrating characteristics of a second excitation light filter provided in the changeover filter;
Fig. 12 is a diagram illustrating characteristics of a third excitation light filter provided in the changeover filter;
Fig. 13 is a diagram illustrating an example of configuration of a rotary filter provided in the light source apparatus;
Fig. 14 is a diagram illustrating characteristics of an optical filter provided in the rotary filter;
Fig. 15 is a diagram illustrating characteristics of an optical filter different from that in Fig. 14 provided in the rotary filter;
Fig. 16 is a diagram illustrating characteristics of an optical filter different from those in Fig. 14 and Fig. 15 provided in the rotary filter;
Fig. 17 is a timing chart illustrating an exposure period and a reading period of a CCD provided in a scope;
Fig. 18 is a timing chart illustrating an insertion operation and a retraction operation of each optical filter accompanying the rotation of the rotary filter;
Fig. 19 is a timing chart illustrating an insertion operation and a retraction operation in a first observation mode of each optical filter provided in the image pickup actuator;
Fig. 20 is a timing chart illustrating an insertion operation and a retraction operation in a second observation mode of each optical filter provided in the image pickup actuator;
Fig. 21 is a timing chart illustrating an insertion operation and a retraction operation in a third observation mode of each optical filter provided in the image pickup actuator;
Fig. 22 is a diagram illustrating an example of image acquired according to a first fluorescent light beam emitted from a first fluorescence probe;
Fig. 23 is a diagram illustrating an example of image acquired according to a second fluorescent light beam emitted from a second fluorescence probe;
Fig. 24 is a diagram illustrating an example of image acquired according to a reference light reflected from an object;
Fig. 25 is a diagram illustrating a synthesized image synthesized from the image in Fig. 22 and the image in Fig. 24;
Fig. 26 is a diagram illustrating an example when the image in Fig. 22 and the image in Fig. 25 are displayed side by side on the same screen;
Fig. 27 is a diagram illustrating a synthesized image synthesized from the image in Fig. 23 and the image in Fig. 24;
Fig. 28 is a diagram illustrating an example when the image in Fig. 23 and the image in Fig. 27 are displayed side by side on the same screen;
Fig. 29 is a diagram illustrating a synthesized image synthesized from the image in Fig. 22 and the image in Fig. 23;
Fig. 30 is a diagram illustrating a synthesized image synthesized from the image in Fig. 22, the image in Fig. 23 and the image in Fig. 24;
Fig. 31 is a diagram illustrating an example when the image in Fig. 29 and the image in Fig. 30 are displayed side by side on the same screen;
Fig. 32 is a diagram illustrating an example when the image in Fig. 22, the image in Fig. 23 and the image in Fig. 24 are displayed side by side on the same screen;
Fig. 33 is a diagram illustrating an example of a brightness detection result of a first image signal, a second image signal and a third image signal;
Fig. 34 is a diagram illustrating an example of a result of adjusting brightness of the first image signal and brightness of the second image signal when the brightness detection result in Fig. 33 is obtained; and
Fig. 35 is a diagram illustrating an example of configuration of a rotary filter provided in the light source apparatus different from that in Fig. 13.

### Best Mode for Carrying Out the Invention

Hereinafter, an embodiment of the present invention will be described with reference to the accompanying drawings.

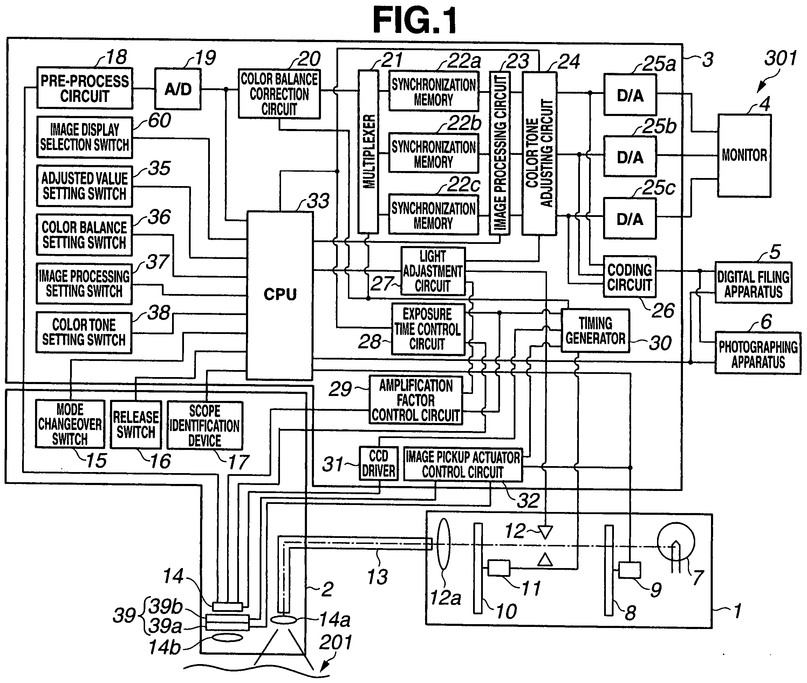

As shown in Fig. 1, an endoscope system 301 is configured by including a scope 2 that can be inserted into a body cavity of a subject, picks up an image of an object 201 in the body cavity and outputs an image pickup signal, a light source apparatus 1 that supplies illuminating light for illuminating the object 201 as an image pickup target of the scope 2, a processor 3 that applies various kinds of signal processing to the image pickup signal from the scope 2 and outputs the signal, a monitor 4 that displays an image corresponding to the output signal from the processor 3, a digital filing apparatus 5 that stores an image corresponding to the output signal from the processor 3 and a photographing apparatus 6 that photographs an image corresponding to the output signal from the processor 3. Furthermore, a light guide 13 for transmitting the illuminating light supplied from the light source apparatus 1 to a distal end portion of the scope 2 is inserted into the scope 2.

The scope 2 is provided, at its distal end portion, with an illumination optical system 14a that emits the illuminating light transmitted by the light guide 13 to the object 201, an objective optical system 14b that forms an image of returning light from the object 201 illuminated with the illuminating light, a monochrome type CCD 14 whose image pickup surface is arranged at an image forming position of the objective optical system 14b and an image pickup actuator 39 arranged on an optical path between the objective optical system 14b and the CCD 14. Furthermore, the scope 2 is provided with a mode changeover switch 15 that allows operation related to switchover between observation modes of the endoscope system 301, a release switch 16 that allows operation related to acquisition of a still image of the object 201 and a scope identification device 17 in which specific identification information corresponding to the type or the like of the scope 2 is stored.

The CCD 14 is driven according to control by the processor 3, applies photoelectrical conversion to returning light from the object 201 whose image is formed on the image pickup surface and thereby generates an image pickup signal and outputs the signal to the processor 3. Furthermore, the CCD 14 of the present embodiment is further provided with an electronic shutter (not shown) that can adjust an exposure time and a reading time according to control by the processor 3. The CCD 14 of the present embodiment is further provided with a charge amplification apparatus (not shown).

Here, a detailed configuration of the image pickup actuator 39 will be described.

A filter changeover apparatus 39a of the image pickup actuator 39 has a configuration capable of switching between a first arrangement state (insertion state) in which a filter that allows to pass only light in a predetermined wavelength band is inserted in an optical path from the objective optical system 14b to the CCD 14 and a second arrangement state (retraction state) in which the filter that allows to pass only the light in the predetermined wavelength band is retracted from the optical path from the objective optical system 14b to the CCD 14 according to control by the processor 3.

To be more specific, the filter changeover apparatus 39a of the image pickup actuator 39 has a configuration similar to the configuration of a light adjuster described in Japanese Patent Application Laid-Open Publication No. 2009-8717. That is, the filter changeover apparatus 39a is configured by including a filter switchover mechanism 101 and a magnet displacement apparatus 102.

The filter switchover mechanism 101 is formed so as to sandwich a filter moving member 105, a closing stopper 107 and an opening stopper 108 between a lower substrate 103 and an upper substrate 104.

One end of a shape memory alloy wire 120 is fixed to a magnet 119 of the magnet displacement apparatus 102. Furthermore, a bias spring 121 and an insulating tube 122 are passed through the shape memory alloy wire 120. On the other hand, another end of the shape memory alloy wire 120 is fixed to a swaging member (not shown). The aforementioned swaging member (not shown) is also fixed at the end on the opposite side of the magnet 119 of the tube 122.

A rotating shaft 109 and a columnar magnet 110 are press-fitted into the filter moving member 105. Furthermore, the filter moving member 105 is provided with an optical filter section 118 having an optical filter 117a.

On the other hand, an optical aperture 111, a rotating shaft insertion hole for inserting the rotating shaft 109 and a notch for guiding the magnet 110 are formed in the lower substrate 103. Furthermore, an optical aperture having a diameter equal to or slightly larger than that of the optical aperture 111, a rotating shaft insertion hole for inserting the rotating shaft 109 and a notch for guiding the magnet 110 are formed in the upper substrate 104 in substantially the same way as for the lower substrate 103.

That is, the rotating shaft 109 is inserted in rotating shaft insertion holes provided in the lower substrate 103 and the upper substrate 104 respectively. This allows the filter moving member 105 to rotate and displace around the rotating shaft 109. The rotatable range of the filter moving member 105 is limited by the closing stopper 107 and the opening stopper 108. Furthermore, the movable range of the magnet 110 is limited by the guide notches provided in the lower substrate 103 and the upper substrate 104 respectively.

According to the above described configuration, when the filter moving member 105 rotates and displaces around the rotating shaft 109, if, for example, the optical filter section 118 contacts the closing stopper 107, a center of the optical filter 117a coincides with a center of the optical aperture 111.

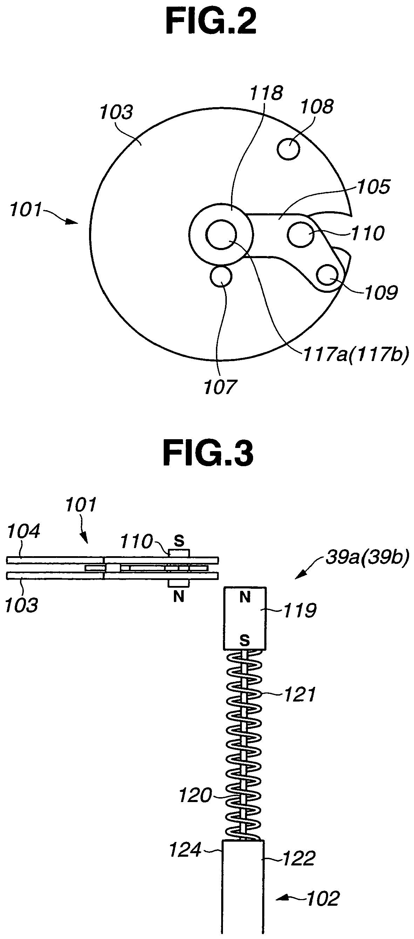

In the aforementioned first arrangement state (insertion state) of the filter changeover apparatus 39a as shown, for example, in Fig. 3, the shape memory alloy wire 120 contracts as a voltage corresponding to control by the processor 3 is applied, the magnet 119 fixed to one end of the shape memory alloy wire 120 displaces toward the tube 122 against a repulsive force of the bias spring 121, and a north pole of the magnet 110 and a north pole of the magnet 119 are thereby arranged facing each other.

In the aforementioned first arrangement state (insertion state), this causes a repulsive force to be generated between the magnet 110 and the magnet 119, which causes the magnet 110 to displace toward a center of the filter switchover mechanism 101. As a result, in the aforementioned first arrangement state (insertion state) as shown, for example, in Fig. 2, the filter moving member 105 rotates around the rotating shaft 109 counterclockwise and the optical filter section 118 contacts the closing stopper 107.

In the aforementioned first arrangement state (insertion state), the optical filter section 118 covers the optical aperture 111, and therefore the filter switchover mechanism 101 allows only returning light of a predetermined wavelength band defined by the optical filter 117a to pass to the image pickup surface of the CCD 14.

On the other hand, according to the above described configuration, when the filter moving member 105 rotates and displaces around the rotating shaft 109, if, for example, the optical filter section 118 contacts the opening stopper 108, the optical filter section 118 is completely retracted from the optical aperture 111.

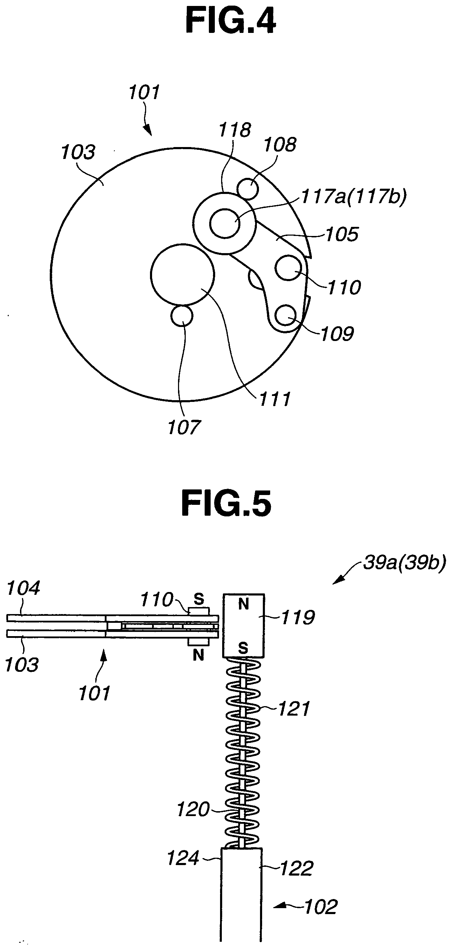

In the aforementioned second arrangement state (retraction state) of the filter changeover apparatus 39a, as shown, for example, in Fig. 5, the shape memory alloy wire 120 extends as a voltage corresponding to control by the processor 3 is applied, the magnet 119 fixed to one end of the shape memory alloy wire 120 displaces toward the opposite side of the tube 122 following a repulsive force of the bias spring 121, and a south pole of the magnet 110 and the north pole of the magnet 119 are arranged facing each other.

In the aforementioned second arrangement state (retraction state), this causes an attractive force to be generated between the magnet 110 and the magnet 119, which causes the magnet 110 to displace toward the circumferential direction of the filter switchover mechanism 101. As a result, in the aforementioned second arrangement state (retraction state), as shown, for example, in Fig. 4, the filter moving member 105 rotates around the rotating shaft 109 clockwise and the optical filter section 118 contacts the opening stopper 108.

In the aforementioned second arrangement state (retraction state), since the optical aperture 111 is not covered with the optical filter section 118, the filter switchover mechanism 101 places no band restriction on the returning light that has passed through the objective optical system 14b and allows the returning light to directly pass toward the image pickup surface of the CCD 14.

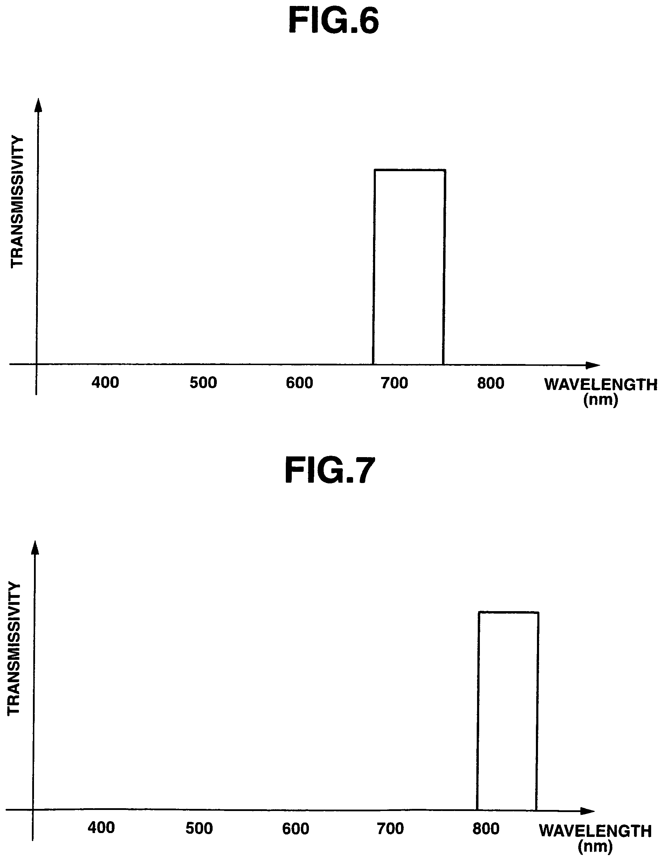

Suppose the optical filter 117a of the filter changeover apparatus 39a in the present embodiment is formed so as to allow to pass only light of 680 to 750 nm as shown, for example, in Fig. 6.

Furthermore, as shown in Fig. 1, the image pickup actuator 39 of the present embodiment is configured by including the filter changeover apparatus 39a and a filter changeover apparatus 39b having a configuration substantially the same as that of the filter changeover apparatus 39a.

The filter changeover apparatus 39b is provided with an optical filter 117b that allows to pass only returning light of a wavelength band different from that of the optical filter 117a, whereas as for the rest of the part, the filter changeover apparatus 39b has the same configuration as that of the filter changeover apparatus 39a. Furthermore, the optical filter 117b is formed so as to allow to pass only light of 790 to 850 nm as shown, for example, in Fig. 7.

As described above, the image pickup actuator 39 of the present embodiment is not limited to the configuration based on the configuration of the light adjuster described in Japanese Patent Application Laid-Open Publication No. 2009-8717.

To be more specific, the image pickup actuator 39 of the present embodiment may also be configured based on other configurations such as a light adjuster described in Japanese Patent Application Laid-Open Publication No. 2009-8719 as long as the optical filters 117a and 117b have a configuration whereby it is possible to switch between the aforementioned first arrangement state (insertion state) and second arrangement state (retraction state).

The light source apparatus 1 is configured by including a lamp 7 that emits light in a wavelength region including a visible region and a near-infrared region, a changeover filter 8 provided so as to vertically traverse an optical path of the lamp 7, a motor 9 that selects one filter to be inserted in the optical path of the lamp 7 from the respective filters of the changeover filter 8, a rotary filter 10 provided so as to vertically traverse the optical path of the lamp 7, a motor 11 that drives the rotary filter 10 to rotate, a diaphragm 12 arranged in the optical path of the lamp 7 from the changeover filter 8 to the rotary filter 10 and a condensing lens 12a that condenses illuminating light that has passed through the rotary filter 10 to an end face of the light guide 13 on the incident light side.

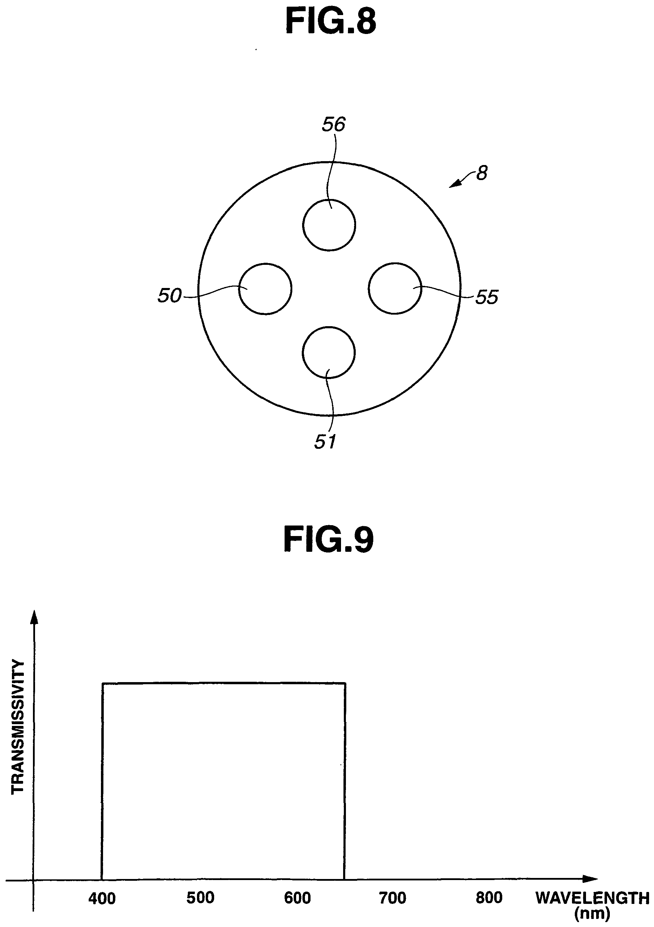

As shown in Fig. 8, the disk-shaped changeover filter 8 is provided with a normal light filter 50 that allows to pass light in a visible region, a first excitation light filter 51 that allows to pass light in part of the visible region and a red color region, a second excitation light filter 55 that allows to pass light in part of the visible region and a near-infrared region and a third excitation light filter 56 that includes both pass bands of the first excitation light filter 51 and the second excitation light filter 55 in a circumferential direction of the disk. That is, the changeover filter 8 is configured such that one of the normal light filter 50, the first excitation light filter 51, the second excitation light filter 55 and the third excitation light filter 56 is inserted in the optical path of the lamp 7 and the remaining three filters other than the one filter are retracted from the optical path of the lamp 7 when the motor 9 rotates according to control by the processor 3.

The normal light filter 50 is formed so as to allow to pass light having a wavelength band of 400 to 650 nm of the light beams in the respective wavelength bands emitted from the lamp 7 as shown in Fig. 9.

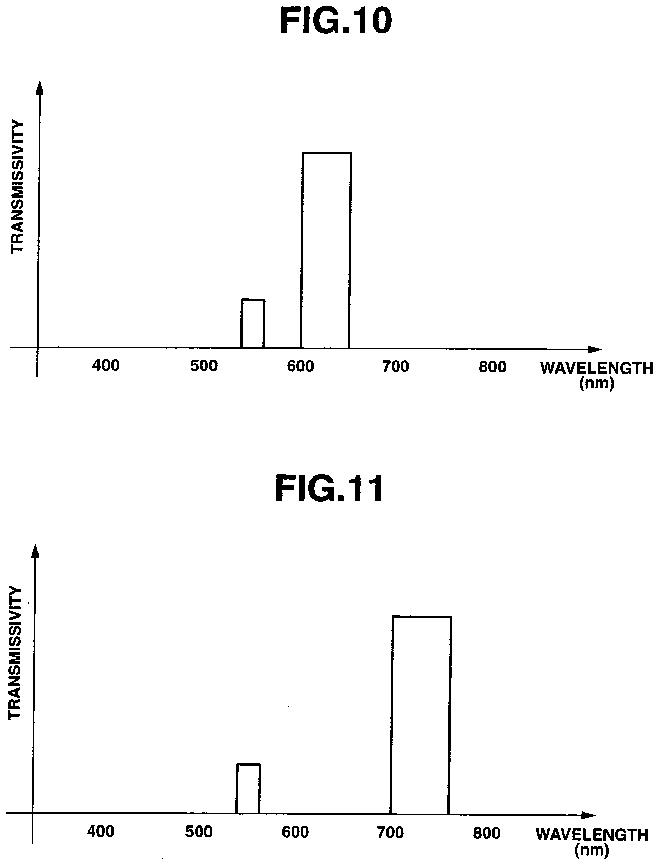

The first excitation light filter 51 is formed so as to allow to pass light having wavelength bands of 540 to 560 nm and 600 to 650 nm of the light beams in the respective wavelength bands emitted from the lamp 7 as shown in Fig. 10.

The second excitation light filter 55 is formed so as to allow to pass light having wavelength bands of 540 to 560 nm and 700 to 760 nm of the light beams in the respective wavelength bands emitted from the lamp 7 as shown in Fig. 11.

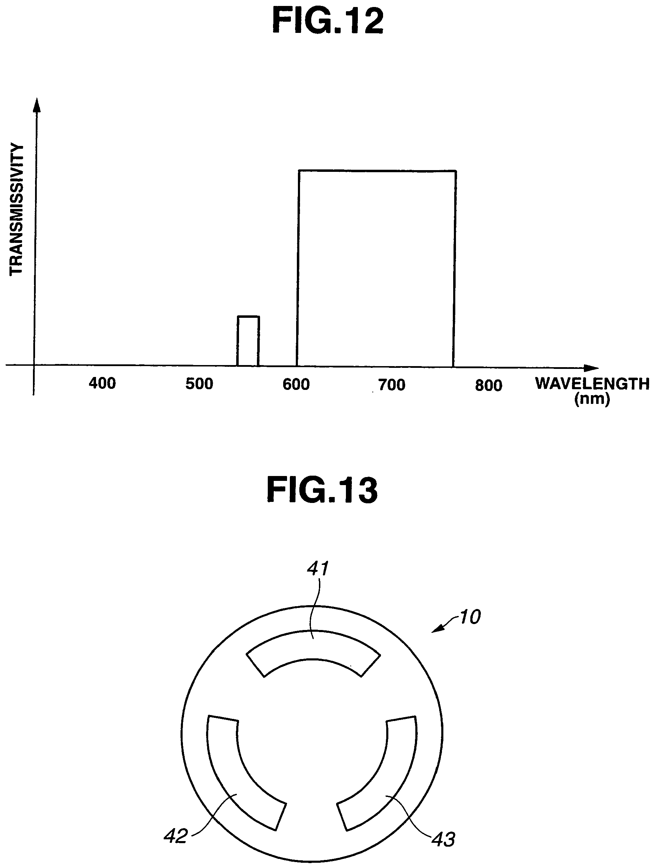

The third excitation light filter 56 is formed so as to allow to pass light having wavelength bands of 540 to 560 nm and 600 to 760 nm of the light beams in the respective wavelength bands emitted from the lamp 7 as shown in Fig. 12.

The diaphragm 12 has a configuration that allows the light quantity of light that has passed through the changeover filter 8 to increase or decrease according to control by the processor 3.

As shown in Fig. 13, the disk-shaped rotary filter 10 is provided with an optical filter 41 that allows to pass light in a red color region, an optical filter 42 that allows to pass light in a green color region, an optical filter 43 that allows to pass light in a blue color region and a near-infrared region along the circumferential direction of the disk. That is, the rotary filter 10 is configured such that the optical filters 41, 42 and 43 are sequentially switched, inserted in the optical path of the lamp 7 or retracted from the optical path of the lamp 7 when the motor 11 rotates according to control by the processor 3 (timing signal of a timing generator 30 which will be described later). Suppose the rotary filter 10 of the present embodiment is formed so as not to allow light to pass when any parts other than the areas in which the optical filters 41, 42 and 43 are arranged are inserted in the optical path of the lamp 7.

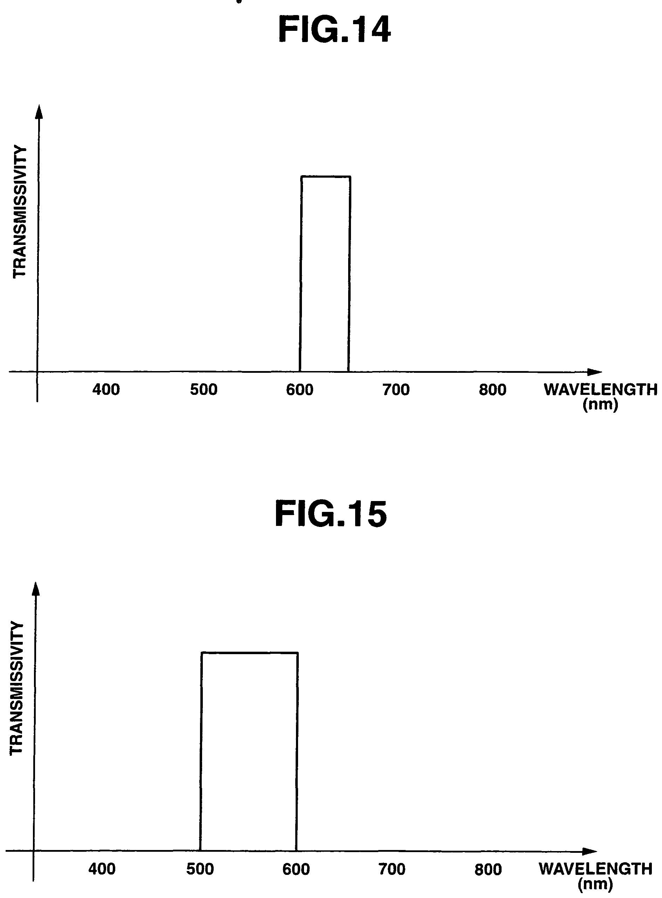

As shown in Fig. 14, the optical filter 41 is formed so as to allow to pass light having a wavelength band of 600 to 650 nm of the respective wavelength bands of the light that has passed through the changeover filter 8 and the diaphragm 12.

As shown in Fig. 15, the optical filter 42 is formed so as to allow to pass light having a wavelength band of 500 to 600 nm of the respective wavelength bands of the light that has passed through the changeover filter 8 and the diaphragm 12.

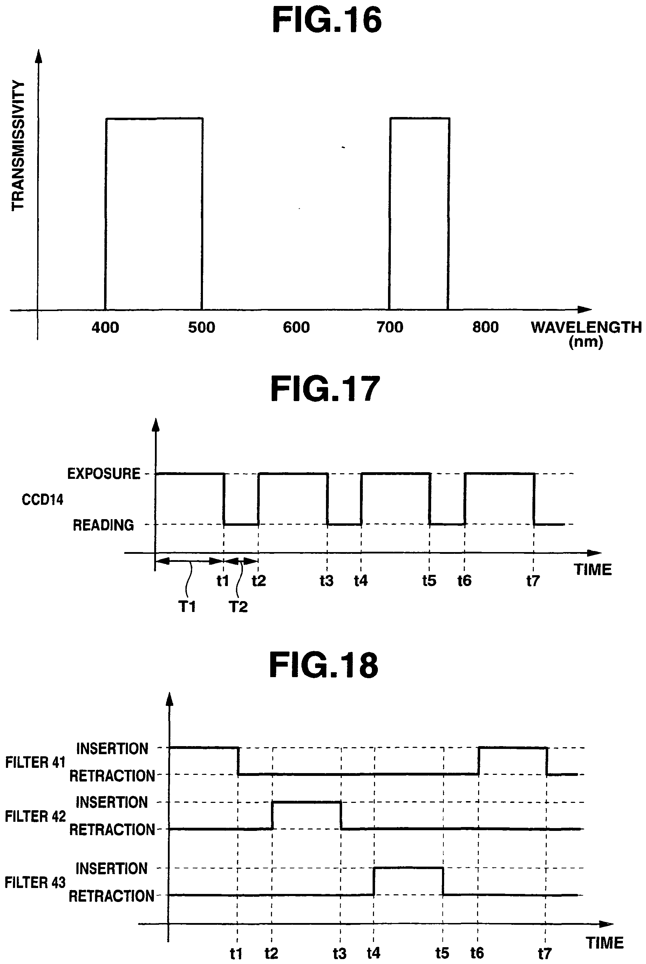

As shown in Fig. 16, the optical filter 43 is formed so as to allow to pass light having wavelength bands of 400 to 500 nm and 700 to 760 nm of the respective wavelength bands of the light that has passed through the changeover filter 8 and the diaphragm 12.

The image pickup signal outputted from the CCD 14 is inputted to the processor 3, then subjected to processing such as CDS (correlation double sampling) at a pre-process circuit 18, converted to a digital image signal at an A/D conversion circuit 19 and then outputted to a color balance correction circuit 20.

The color balance correction circuit 20 selects color balance correction coefficients corresponding to the optical filters 41, 42 and 43 so as to synchronize with timing at which the optical filters 41, 42 and 43 of the rotary filter 10 are sequentially inserted in the optical path of the lamp 7 based on timing signals from the timing generator 30 and reads a selected color balance correction coefficients from a memory (not shown). The color balance correction circuit 20 then multiplies image signals sequentially outputted from the A/D conversion circuit 19 by the color balance correction coefficients read from the memory (not shown) and then outputs the multiplied image signals to a multiplexer 21.

The aforementioned color balance correction coefficients are correction values calculated through calculation processing by a CPU 33 in color balance operation such as white balance and are stored in the memory (not shown) of the color balance correction circuit 20 as processing results of the calculation processing. Furthermore, the color balance operation such as the aforementioned white balance is started by a color balance setting switch 36 provided in the processor 3 at timing at which the CPU 33 detects operation related to the start of execution of the color balance operation.

The multiplexer 21 distributes and outputs the image signal outputted from the color balance correction circuit 20 to synchronization memories 22a, 22b and 22c as appropriate so as to synchronize with timing at which the optical filters 41, 42 and 43 are sequentially inserted in the optical path of the lamp 7 based on the timing signals from the timing generator 30.

The synchronization memories 22a, 22b and 22c have a configuration capable of temporarily storing the image signals outputted from the multiplexer 21.

An image processing circuit 23 simultaneously reads the image signals stored in the synchronization memories 22a, 22b and 22c and then applies predetermined image processing to the three read image signals. The image processing circuit 23 then assigns the three image signals after the predetermined image processing to a first color channel corresponding to a first color component (e.g., red (R) component), a second color channel corresponding to a second color component (e.g., green (G) component) and a third color channel corresponding to a third color component (e.g., blue (B) component) and outputs the image signals to a color tone adjusting circuit 24.

The color tone adjusting circuit 24 reads color tone adjustment coefficients stored in a memory (not shown) and then performs matrix calculation processing using the color tone adjustment coefficients, the image signal of the first color component (first color channel) outputted from the image processing circuit 23, the image signal of the second color component (second color channel) and the image signal of the third color component (third color channel). After that, the color tone adjusting circuit 24 applies gamma correction processing to the image signal of the first color component, the image signal of the second color component and the image signal of the third color component after applying the aforementioned matrix calculation processing. The color tone adjusting circuit 24 outputs the image signals of the first color component, the second color component and the third color component after applying the aforementioned gamma correction processing to a coding circuit 26 and a light adjustment circuit 27 respectively. Furthermore, the color tone adjusting circuit 24 outputs the image signal of the first color component after applying the aforementioned gamma correction processing to a D/A conversion circuit 25a, outputs the image signal of the second color component to a D/A conversion circuit 25b and outputs the image signal of the third color component to a D/A conversion circuit 25c.

The aforementioned color tone adjustment coefficients are adjusted values calculated through calculation processing by the CPU 33 in a color tone adjustment operation and stored in a memory (not shown) of the color tone adjusting circuit 24 as a processing result of the calculation processing. Furthermore, the aforementioned color tone adjustment operation is started by a color tone setting switch 38 provided in the processor 3 at timing at which the CPU 33 detects operation related to a change of color tone displayed on the monitor 4. The CPU 33 then performs calculation processing to calculate a color tone adjustment coefficient corresponding to the changed color tone when the operation related to the change of color tone displayed on the monitor 4 is performed.

The image signals of the first color component, the second color component and the third color component outputted from the color tone adjusting circuit 24 are converted to analog video signals at the D/A conversion circuits 25a, 25b and 25c respectively and then outputted to the monitor 4. Thus, the monitor 4 displays an observation image corresponding to each observation mode.

Furthermore, the image signals of the first color component, the second color component and the third color component outputted from the color tone adjusting circuit 24 are subjected to coding processing at the coding circuit 26 respectively and then outputted to the digital filing apparatus 5 and the photographing apparatus 6. Thus, when the CPU 33 detects an input operation by the release switch 16, the digital filing apparatus 5 records a still image as image data. Furthermore, when the CPU 33 detects an input operation by the release switch 16, the photographing apparatus 6 takes a still image.

The light adjustment circuit 27 performs control on the diaphragm 12 so that an appropriate quantity of illuminating light corresponding to the observation mode is supplied from the light source apparatus 1 based on the respective signal levels of the image signals of the first color component, the second color component and the third color component outputted from the color tone adjusting circuit 24. Furthermore, the light adjustment circuit 27 performs control of changing an amplification factor of an amplification factor control circuit 29.

Based on timing signals outputted from the timing generator 30 and output signals from the CPU 33, an exposure time control circuit 28 controls the electronic shutter of the CCD 14 so as to synchronize with timing at which the optical filters 41, 42 and 43 are sequentially inserted in the optical path of the lamp 7 and correspond to the output signals from the CPU 33. Exposure times by the CCD 14 are changed through such control on the electronic shutter.

Based on the control by the light adjustment circuit 27 and timing signals outputted from the timing generator 30, the amplification factor control circuit 29 controls the charge amplification apparatus of the CCD 14 so as to synchronize with timing at which the optical filters 41, 42 and 43 are sequentially inserted in the optical path of the lamp 7 and obtain an amplification factor that responds to the control of the light adjustment circuit 27. The amplification factor in the CCD 14 is changed through such control on the charge amplification apparatus.

The timing generator 30 generates and outputs timing signals for synchronizing operations of the respective sections of the endoscope system 301 appropriately.

A CCD driver 31 drives the CCD 14 so as to be synchronized with timing at which the optical filters 41, 42 and 43 are sequentially inserted in the optical path of the lamp 7 based on timing signals outputted from the timing generator 30.

Based on timing signals outputted from the timing generator 30, an image pickup actuator control circuit 32 performs control on the image pickup actuator 39 for synchronizing between timing at which the optical filters 41, 42 and 43 are sequentially inserted in the optical path of the lamp 7, timing of switchover between arrangement states of the optical filter 117a in the filter changeover apparatus 39a and timing of switchover between arrangement states of the optical filter 117b of the filter changeover apparatus 39b.

The CPU 33 detects operation states in an adjusted value setting switch 35, the color balance setting switch 36, an image processing setting switch 37 and the color tone setting switch 38 provided in the processor 3 and performs control and processing or the like according to the detection results.

The CPU 33 detects an operation state of an image display selection switch 60 provided in the processor 3 and performs control on the image processing circuit 23 for causing an observation image according to the detection result to be outputted to the monitor 4.

The CPU 33 detects an operation state of the mode changeover switch 15 of the scope 2 connected to the processor 3 and performs control on the motor 9 or the like of the light source apparatus 1 for changing the mode to an observation mode according to the detection result.

The CPU 33 detects an operation state of the release switch 16 of the scope 2 connected to the processor 3 and performs control related to recording of a still image in the digital filing apparatus 5 and (or) image taking of a still image in the photographing apparatus 6 according to the detection result.

When the scope 2 is connected to the processor 3, the CPU 33 reads information stored in the scope identification device 17 and performs control according to the read information.

Suppose the CPU 33 of the present embodiment is connected to the respective sections of the processor 3 via signal lines (not shown) or the like so as to be able to perform comprehensive control on the respective sections of the processor 3.

Next, operations of the present embodiment will be described.

First, a surgeon or the like connects the respective sections of the endoscope system 301, turns on power to start operations of the respective sections.

On the other hand, upon powering on of the processor 3, the timing generator 30 starts to output timing signals.

The CCD driver 31 drives the CCD 14 according to, for example, a timing chart in Fig. 17 based on the timing signals from the timing generator 30. Thus, the CCD 14 operates in such a way that an exposure period T1 as a period related to accumulation of charge and a reading period T2 as a period related to discharging of the charge accumulated for the exposure period T1 are switched alternately.

Furthermore, when power is supplied to the light source apparatus 1 and the timing generator 30 starts to output timing signals, the motor 11 starts to be driven to rotate. When the motor 11 is driven to rotate, the optical filters 41, 42 and 43 are sequentially switched, inserted in the optical path of the lamp 7 or retracted from the optical path of the lamp 7. The insertion operation and the retraction operation of the optical filters 41, 42 and 43 caused by the rotation and drive of the motor 11 are performed at timing corresponding to a timing chart in Fig. 18. That is, the motor 11 causes the rotary filter 10 to rotate so that the optical filters 41, 42 and 43 are sequentially inserted in the optical path of the lamp 7 for an exposure period of the CCD 14 and the optical filters 41, 42 and 43 are retracted from the optical path of the lamp 7 for a reading period of the CCD 14.

On the other hand, the surgeon or the like operates the mode changeover switch 15 of the scope 2 and thereby instructs the endoscope system 301 to transition to a desired observation mode. Furthermore, the surgeon or the like administers or scatter a first fluorescence probe provided with an excitation wavelength of 600 to 650 nm and a fluorescent light wavelength of 680 to 750 nm and a second fluorescence probe provided with an excitation wavelength of 700 to 760 nm and a fluorescent wavelength of 790 to 850 nm beforehand using the scope 2 before performing observation of the object 201.

Here, in the present embodiment, the mode changeover switch 15 can make a changeover to four observation modes corresponding to the number of filters provided in the changeover filter 8.

For example, when an operation related to a selection of a first observation mode is performed with the mode changeover switch 15, the CPU 33 controls the motor 9 of the light source apparatus 1 to thereby cause the first excitation light filter 51 to be inserted in the optical path of the lamp 7. That is, in the aforementioned first observation mode, a frame-sequential first illuminating light including a reference light having a wavelength band of 540 to 560 nm and a first excitation light having a wavelength band of 600 to 650 nm is supplied to the light guide 13.

Furthermore, when an operation related to a selection of the first observation mode is performed with the mode changeover switch 15, the image pickup actuator control circuit 32 operates the image pickup actuator 39 based on the control by the CPU 33 so as to synchronize timing at which the optical filters 41, 42 and 43 are sequentially inserted in the optical path of the lamp 7 with timing at which the filter changeover apparatus 39a changes the arrangement state of the optical filter 117a.

To be more specific, as shown in Fig. 17, Fig. 18 and Fig. 19, in the aforementioned first observation mode, the image pickup actuator control circuit 32 sets the arrangement state of the optical filter 117a of the filter changeover apparatus 39a to the aforementioned first arrangement state (insertion state) and further sets the arrangement state of the optical filter 117b of the filter changeover apparatus 39b to the aforementioned second arrangement state (retraction state) for an exposure period of the CCD 14 and a period during which the optical filter 41 is inserted in the optical path of the lamp 7. On the other hand, as shown in Fig. 17, Fig. 18 and Fig. 19, in the aforementioned first observation mode, the image pickup actuator control circuit 32 sets the arrangement state of the optical filter 117a of the filter changeover apparatus 39a to the aforementioned second arrangement state (retraction state) and further sets the arrangement state of the optical filter 117b of the filter changeover apparatus 39b to the aforementioned second arrangement state (retraction state) for a reading period of the CCD 14 and a period during which the optical filter 42 is inserted in the optical path of the lamp 7 or a period during which the optical filter 43 is inserted in the optical path of the lamp 7.

Therefore, in the aforementioned first observation mode, since the first fluorescence probe is excited by the first illuminating light (first excitation light) emitted from the light guide 13, images of the first fluorescent light having a wavelength band of 680 to 750 nm and the reference light having a wavelength band of 540 to 560 nm are sequentially formed on the image pickup surface of the CCD 14 as returning light from the object 201.

When, for example, an operation related to a selection of a second observation mode is performed with the mode changeover switch 15, the CPU 33 controls the motor 9 of the light source apparatus 1 and thereby causes the second excitation light filter 55 to be inserted in the optical path of the lamp 7. That is, in the aforementioned second observation mode, a frame-sequential second illuminating light including the reference light having a wavelength band of 540 to 560 nm and a second excitation light having a wavelength band of 700 to 760 nm is supplied to the light guide 13.

Furthermore, when an operation related to a selection of the second observation mode is performed with the mode changeover switch 15, the image pickup actuator control circuit 32 operates the image pickup actuator 39 so as to synchronize timing at which the optical filters 41, 42 and 43 are sequentially inserted in the optical path of the lamp 7 with timing at which the filter changeover apparatus 39b changes the arrangement state of the optical filter 117b based on the control by the CPU 33.

To be more specific, as shown in Fig. 17, Fig. 18 and Fig. 20, in the aforementioned second observation mode, the image pickup actuator control circuit 32 sets the arrangement state of the optical filter 117a of the filter changeover apparatus 39a to the aforementioned second arrangement state (retraction state) and further sets the arrangement state of the optical filter 117b of the filter changeover apparatus 39b to the aforementioned first arrangement state (insertion state) for an exposure period of the CCD 14 and for a period during which the optical filter 43 is inserted in the optical path of the lamp 7. On the other hand, as shown in Fig. 17, Fig. 18 and Fig. 20, in the aforementioned second observation mode, the image pickup actuator control circuit 32 sets the arrangement state of the optical filter 117a of the filter changeover apparatus 39a to the aforementioned second arrangement state (retraction state) and further sets the arrangement state of the optical filter 117b of the filter changeover apparatus 39b to the aforementioned second arrangement state (retraction state) for a reading period of the CCD 14 and for a period during which the optical filter 41 is inserted in the optical path of the lamp 7 or for a period during which the optical filter 42 is inserted in the optical path of the lamp 7.

Therefore, in the aforementioned second observation mode, since the second fluorescence probe is excited by the second illuminating light (second excitation light) emitted from the light guide 13, images of a second fluorescent light having a wavelength band of 790 to 850 nm and the reference light having a wavelength band of 540 to 560 nm are sequentially formed on the image pickup surface of the CCD 14 as returning light from the object 201.

When, for example, an operation related to a selection of a third observation mode is performed with the mode changeover switch 15, the CPU 33 controls the motor 9 of the light source apparatus 1 and thereby causes the third excitation light filter 56 to be inserted in the optical path of the lamp 7. That is, in the third observation mode, a frame-sequential third illuminating light including the reference light having a wavelength band of 540 to 560 nm, the first excitation light having a wavelength band of 600 to 650 nm and the second excitation light having a wavelength band of 700 to 760 nm is supplied to the light guide 13.

Furthermore, when an operation related to a selection of the third observation mode is performed with the mode changeover switch 15, the image pickup actuator control circuit 32 operates the image pickup actuator 39 so as to synchronize timing at which the optical filters 41, 42 and 43 are sequentially inserted in the optical path of the lamp 7, timing at which the filter changeover apparatus 39a changes the arrangement state of the optical filter 117a and timing at which the filter changeover apparatus 39b changes the arrangement state of the optical filter 117b based on the control by the CPU 33.

To be more specific, as shown in Fig. 17, Fig. 18 and Fig. 21, in the aforementioned third observation mode, the image pickup actuator control circuit 32 sets the arrangement state of the optical filter 117a of the filter changeover apparatus 39a to the aforementioned first arrangement state (insertion state) and further sets the arrangement state of the optical filter 117b of the filter changeover apparatus 39b to the aforementioned second arrangement state (retraction state) for an exposure period of the CCD 14 and for a period during which the optical filter 41 is inserted in the optical path of the lamp 7. Furthermore, as shown in Fig. 17, Fig. 18 and Fig. 21, in the aforementioned third observation mode, the image pickup actuator control circuit 32 sets the arrangement state of the optical filter 117a of the filter changeover apparatus 39a to the aforementioned second arrangement state (retraction state) and further sets the arrangement state of the optical filter 117b of the filter changeover apparatus 39b to the aforementioned first arrangement state (insertion state) for an exposure period of the CCD 14 and for a period during which the optical filter 43 is inserted in the optical path of the lamp 7. On the other hand, as shown in Fig. 17,

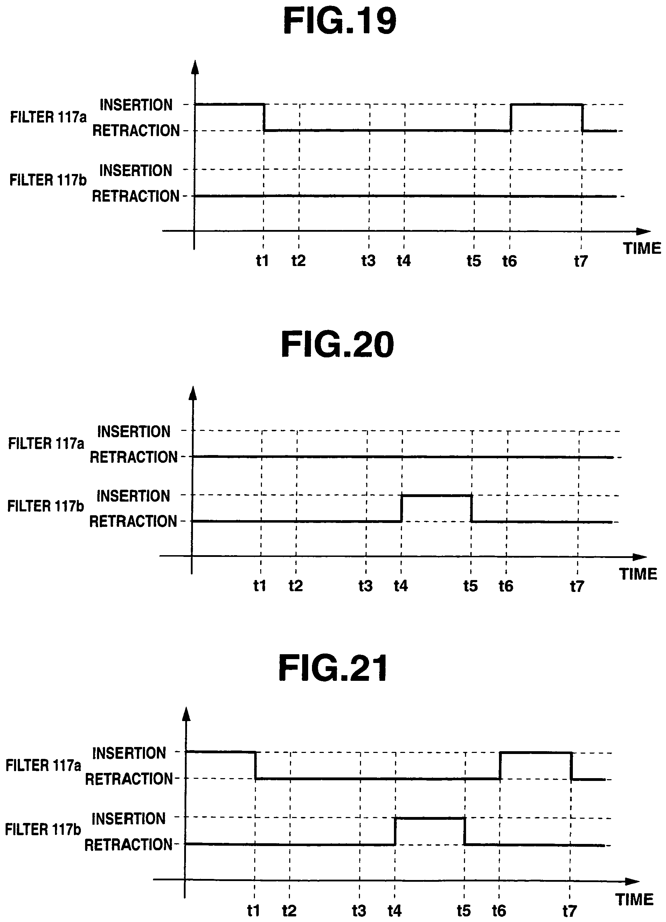

Fig. 18 and Fig. 21, in the aforementioned third observation mode, the image pickup actuator control circuit 32 sets the arrangement state of the optical filter 117a of the filter changeover apparatus 39a to the aforementioned second arrangement state (retraction state) and further sets the arrangement state of the optical filter 117b of the filter changeover apparatus 39b to the aforementioned second arrangement state (retraction state) for a reading period of the CCD 14 or for a period during which the optical filter 42 is inserted in the optical path of the lamp 7.

Therefore, in the aforementioned third observation mode, since the first fluorescence probe and the second fluorescence probe are excited by the third illuminating light (the first excitation light and the second excitation light) emitted from the light guide 13, images of the first fluorescent light having a wavelength band of 680 to 750 nm, the second fluorescent light having a wavelength band of 790 to 850 nm and the reference light having a wavelength band of 540 to 560 nm are sequentially formed on the image pickup surface of the CCD 14 as returning light from the object 201.

Furthermore, when an operation related to a selection of a fourth observation mode is performed with the mode changeover switch 15, the CPU 33 controls the motor 9 of the light source apparatus 1 and thereby causes the normal light filter 50 to be inserted in the optical path of the lamp 7. That is, in the fourth observation mode, a frame-sequential fourth illuminating light including red color light (R light) having a wavelength band of 600 to 650 nm, green color light (G light) having a wavelength band of 500 to 600 nm and blue color light (B light) having a wavelength band of 400 to 500 nm is supplied to the light guide 13.

Furthermore, when an operation related to a selection of the fourth observation mode is performed with the mode changeover switch 15, the image pickup actuator control circuit 32 sets the arrangement state of the optical filter 117a of the filter changeover apparatus 39a and the arrangement state of the optical filter 117b of the filter changeover apparatus 39b to the aforementioned second arrangement state (retraction state) based on the control by the CPU 33.

Therefore, in the aforementioned fourth observation mode, images of reflected light of the fourth illuminating light (R light, G light and B light) emitted from the light guide 13 are sequentially formed on the image pickup surface of the CCD 14 as returning light from the object 201.

Hereinafter, a case where the aforementioned third observation mode is selected by the mode changeover switch 15 will be mainly described.

In the aforementioned third observation mode, image pickup signals corresponding to the first fluorescent light, the second fluorescent light and the reference light are sequentially outputted from the CCD 14. The respective image pickup signals sequentially outputted from the CCD 14 are passed through the pre-process circuit 18, the A/D conversion circuit 19, the color balance correction circuit 20 and the multiplexer 21, and then stored in the synchronization memories 22a, 22b and 22c respectively.

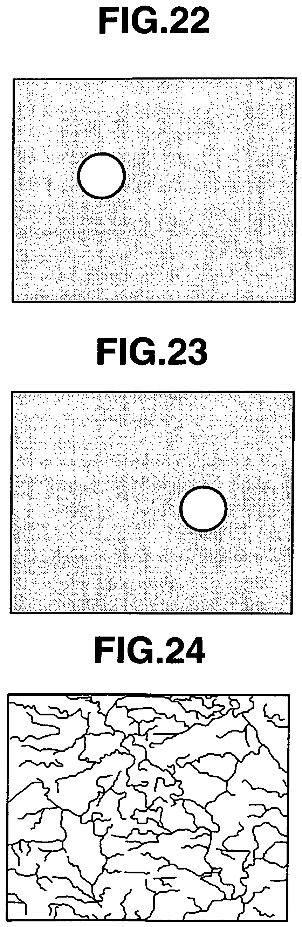

Of the image signals outputted from the multiplexer 21 in the aforementioned third observation mode, an image related to the first image signal corresponding to the first fluorescent light is as shown, for example, in Fig. 22. Furthermore, of the image signals outputted from the multiplexer 21 in the aforementioned third observation mode, an image related to the second image signal corresponding to the second fluorescent light is as shown, for example, in Fig. 23. Furthermore, of the image signals outputted from the multiplexer 21 in the aforementioned third observation mode, an image related to the third image signal corresponding to the reference light is as shown, for example, in Fig. 24.

Here, in the present embodiment, it is possible to change the display mode of observation images displayed on the monitor 4 in various ways through the operation of an image display selection switch 60.

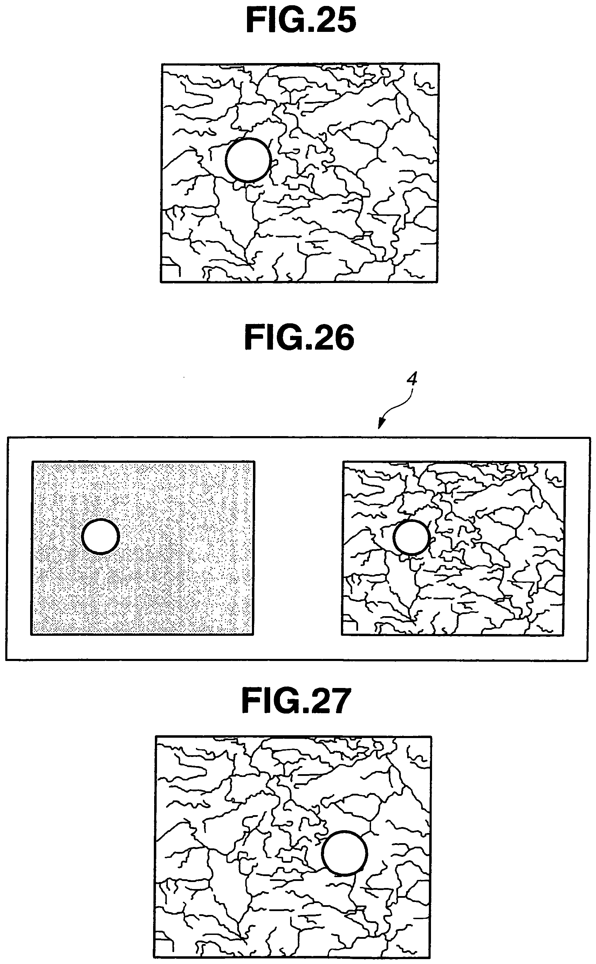

When, for example, an operation related to a selection of a first display mode is performed with the image display selection switch 60, the CPU 33 controls the image processing circuit 23, and thereby generates a synthesized image in Fig. 25 synthesized from the image in Fig. 22 and the image in Fig. 24 assigned to different color channels of the first to the third color channels (R, G and B channels). The CPU 33 then controls the image processing circuit 23 and thereby performs control such that the monochrome image in Fig. 22 and the synthesized image in Fig. 25 are displayed side by side on the same screen. Thus, an observation image in the first display mode as shown in Fig. 26 is displayed on the monitor 4. According to the observation image in the first display mode in Fig. 26, the surgeon or the like can perform observation while comparing information related to a part where the first fluorescence probe is integrated and information related to the structure of the object 201.

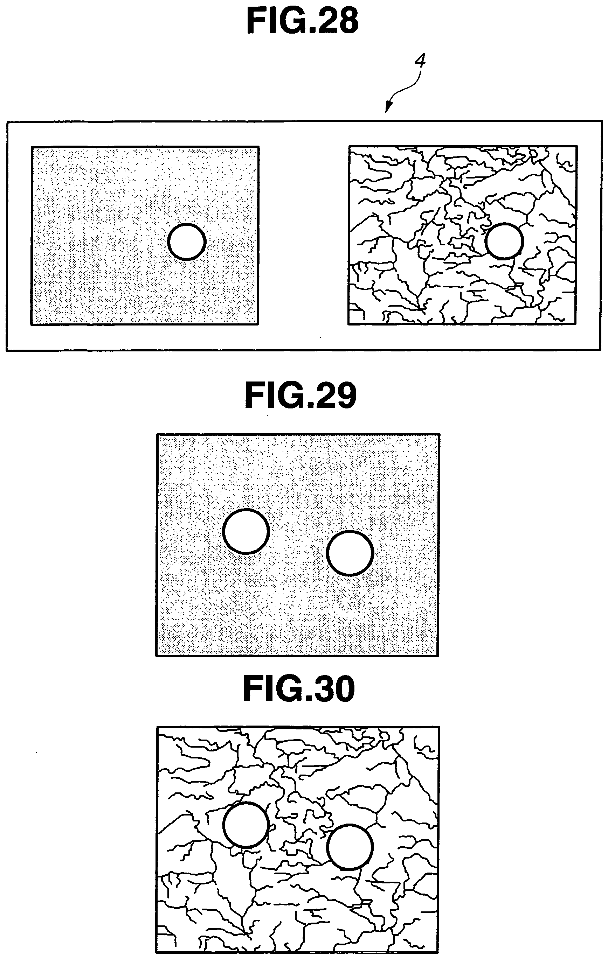

For example, when an operation related to a selection of a second display mode is performed with the image display selection switch 60, the CPU 33 controls the image processing circuit 23 and thereby generates a synthesized image in Fig. 27 synthesized from the image in Fig. 23 and the image in Fig. 24 assigned to different color channels of the first to the third color channels (R, G and B channels). The CPU 33 then controls the image processing circuit 23 and thereby performs control such that the monochrome image in Fig. 23 and the synthesized image in Fig. 27 are displayed side by side on the same screen. Thus, an observation image in the second display mode as shown in Fig. 28 is displayed on the monitor 4. According to the observation image in the second display mode in Fig. 28, the surgeon or the like can perform observation while comparing information related to a part where the second fluorescence probe is integrated and information related to the structure of the object 201.

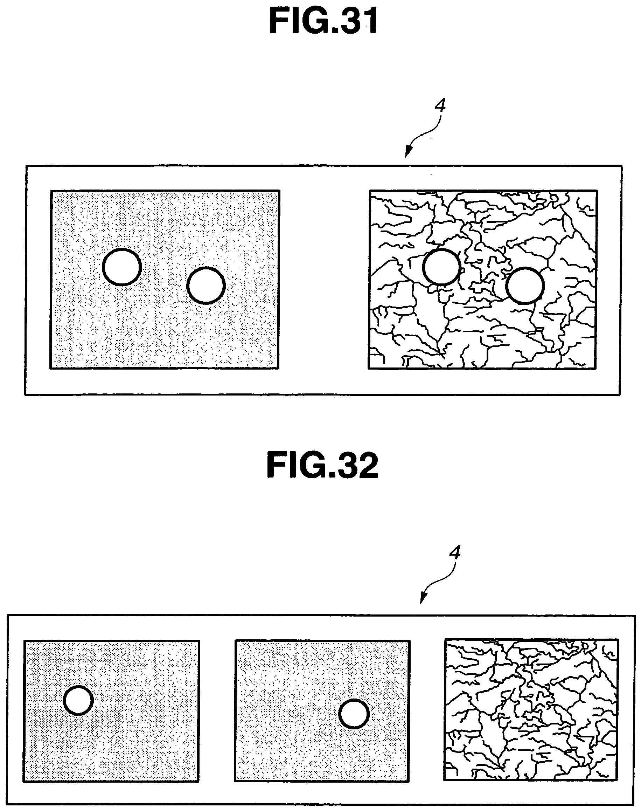

When, for example, an operation related to a selection of a third display mode is performed with the image display selection switch 60, the CPU 33 controls the image processing circuit 23 and thereby generates a synthesized image in Fig. 29 synthesized from the image in Fig. 22 and the image in Fig. 23 assigned to different color channels of the first to the third color channels (R, G and B channels) and further generates a synthesized image in Fig. 30 synthesized from the image in Fig. 22, the image in Fig. 23 and the image in Fig. 24 assigned to different color channels of the first to the third color channels (R, G and B channels). The CPU 33 then controls the image processing circuit 23 and thereby performs control such that the synthesized image in Fig. 29 and the synthesized image in Fig. 30 are displayed side by side on the same screen. Thus, an observation image in the third display mode as shown in Fig. 31 is displayed on the monitor 4. According to the observation image in the third display mode in Fig. 31, the surgeon or the like can perform observation while comparing information related to a part where the first and the second fluorescence probes are integrated and information related to the structure of the object 201.

The present embodiment is not limited to the display mode in which two images are arranged side by side, but a display mode in which three or more images are arranged side by side as shown, for example, in Fig. 32 as long as an image resulting from synthesizing the images in Fig. 22, Fig. 23 and Fig. 24, one each, or a plurality thereof is displayed. The observation image shown in Fig. 32 illustrates an example where the image in Fig. 22, the image in Fig. 23 and the image in Fig. 24 are displayed side by side on the same screen of the monitor 4.

Furthermore, the present embodiment can individually change color tones of a portion corresponding to the image in Fig. 22, a portion corresponding to the image in Fig. 23 and a portion corresponding to the image in Fig. 24 of the observation image displayed on the monitor 4 according to the operation of the color tone setting switch 38.

When, for example, the surgeon or the like performs an operation of individually changing color tones of a portion corresponding to the image in Fig. 22, a portion corresponding to the image in Fig. 23 and a portion corresponding to the image in Fig. 24 of the observation image displayed on the monitor 4 to desired color tones using the color tone setting switch 38, the CPU 33 performs calculation processing for calculating color tone adjustment coefficients according to the desired color tones.

The CPU 33 calculates coefficients used for matrix calculation processing in the color tone adjusting circuit 24 as the aforementioned color tone adjustment coefficients and stores the calculation results in a memory (not shown) of the color tone adjusting circuit 24.

The color tone adjusting circuit 24 performs matrix calculation processing using the color tone adjustment coefficients stored in the memory (not shown), an image signal of the first color component (first color channel) corresponding to, for example, the image in Fig. 22, an image signal of the second color component (second color channel) corresponding to, for example, the image in Fig. 24 and an image signal of the third color component (third color channel) corresponding to, for example, the image in Fig. 23. Thus, it is possible to adjust the color tones of a portion corresponding to the image in Fig. 22, a portion corresponding to the image in Fig. 23 and a portion corresponding to the image in Fig. 24 of the observation image displayed on the monitor 4 to the surgeon's desired color tones.

In conjunction with the operation of the color tone setting switch 38, the present embodiment may also display on the monitor 4 information for notifying the surgeon or the like of the color tones in which the portion corresponding to the image in Fig. 22, the portion corresponding to the image in Fig. 23 and the portion corresponding to the image in Fig. 24 of the observation image displayed on the monitor 4 are currently displayed respectively.

Depending on the type of a fluorescence probe acting on the object 201, there can be a situation in which, for example, the brightness of the image in Fig. 22 as an image corresponding to the first fluorescent light may be too bright or too dark with respect to the brightness of the image in Fig. 24 as an image corresponding to the reference light. Likewise, there can also be a situation in which, for example, the brightness of the image in Fig. 23 as an image corresponding to the second fluorescent light may be too bright or too dark with respect to the brightness of the image in Fig. 24 as an image corresponding to the reference light.

Therefore, the endoscope system 301 of the present embodiment adjusts the brightness of the first image signal corresponding to the first fluorescent light and the brightness of the second image signal corresponding to the second fluorescent light respectively using the brightness of the third image signal corresponding to the reference light as a reference. A more specific example of processing or the like carried out in such an adjustment of brightness will be described below. Parameters of brightness to be adjusted in the processing or the like, which will be described below, may be any one of an average value of signal levels of an image signal, a peak value of signal levels of an image signal or the like.

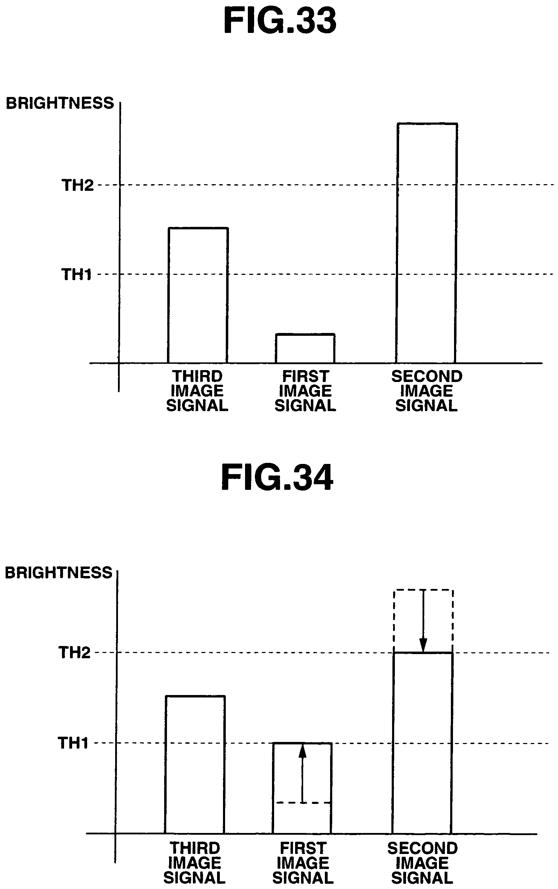

The CPU 33 reads the first image signal corresponding to the first fluorescent light, the second image signal corresponding to the second fluorescent light and the third image signal corresponding to the reference light from the image processing circuit 23 and detects the brightness of each read image signal. In this way, the CPU 33 obtains a detection result as shown, for example, in Fig. 33.

Furthermore, the CPU 33 reads brightness adjusted values stored in a memory (not shown) before and after detecting the brightness of each of the aforementioned image signals. Furthermore, the CPU 33 sets a value obtained by subtracting the aforementioned brightness adjusted value from the brightness of the image corresponding to the reference light as a brightness lower limit value TH1 and sets a value obtained by adding the aforementioned brightness adjusted value to the brightness of the image corresponding to the reference light as a brightness upper limit value TH2. The CPU 33 then determines whether or not the brightness of the first image signal and the brightness of the second image signal are equal to or above the lower limit value TH1 and equal to or below the upper limit value TH2.

In the present embodiment, the lower limit value TH1 and the upper limit value TH2 may be predetermined fixed values or may be changed to desired values for the surgeon or the like according to the operation of the adjusted value setting switch 35. The lower limit value TH1 and the upper limit value TH2 may be set as common values among the respective image signals or may be set as individual values for the respective image signals.

When, for example, the detection result illustrated in Fig. 33 further shows that the brightness of the first image signal is less than the lower limit value TH1, the CPU 33 performs calculation processing for calculating such an adjusted value that the brightness of the first image signal is equal to or above the lower limit value TH1.

Furthermore, the detection result illustrated in Fig. 33 further shows that the brightness of the second image signal exceeds the upper limit value TH2, the CPU 33 performs calculation processing for calculating such an adjusted value that the brightness of the second image signal is equal to or below the upper limit value TH2.

Furthermore, the CPU 33 calculates, for example, color tone adjustment coefficients used for matrix calculation processing as such adjusted values that the brightness of the first image signal becomes the lower limit value TH1 or above and the brightness of the second image signal becomes the upper limit value TH2 or below and outputs the color tone adjustment coefficients to the color tone adjusting circuit 24. In this case, the color tone adjusting circuit 24 performs matrix calculation processing using the color tone adjustment coefficients outputted from the CPU 33, the image signal of the first color component (first color channel) outputted from the image processing circuit 23, the image signal of the second color component (second color channel) and the image signal of the third color component (third color channel).

Alternatively, the CPU 33 calculates, for example, control parameters used to control the electronic shutter as such adjusted values that the brightness of the first image signal becomes equal to or above the lower limit value TH1 and the brightness of the second image signal becomes equal to or below the upper limit value TH2 and outputs the control parameters to the exposure time control circuit 28. In this case, the exposure time control circuit 28 controls the electronic shutter of the CCD 14 based on the timing signals outputted from the timing generator 30 and control parameters outputted from the CPU 33 and thereby adjusts the exposure time in the CCD 14.

When the above described processing or the like is performed, as shown, for example, in Fig. 34, the brightness of the first image signal is increased to the lower limit value TH1 or above and the brightness of the second image signal is decreased to the upper limit value TH2 or below. Thus, for example, even when the synthesized image as shown in Fig. 30 is generated and outputted to the monitor 4, the portion in which the first fluorescence probe is integrated and the portion in which the second fluorescence probe is integrated are displayed with brightness appropriate for a diagnosis respectively.

In the above described processing or the like, the brightness adjusted values stored in the memory (not shown) may be predetermined fixed values or can be changed to desired values for the surgeon or the like according to the operation of the adjusted value setting switch 35. Furthermore, in the above described processing or the like, when, for example, the aforementioned brightness adjusted value is set to 0, it is also possible to adjust the brightness of the first image signal and the brightness of the second image signal to the same brightness as the brightness of the third image signal.

The CCD 14 of the present embodiment may also be configured as a color CCD in which color filters (not shown) are arranged on the image pickup surface.

In such a configuration, upon detecting that the aforementioned fourth observation mode is selected by the mode changeover switch 15, the CPU 33 performs control on the motor 9 for inserting the normal light filter 50 in the optical path of the lamp 7 and also performs control on the motor 11 for retracting the rotary filter 10 from the optical path of the lamp 7.

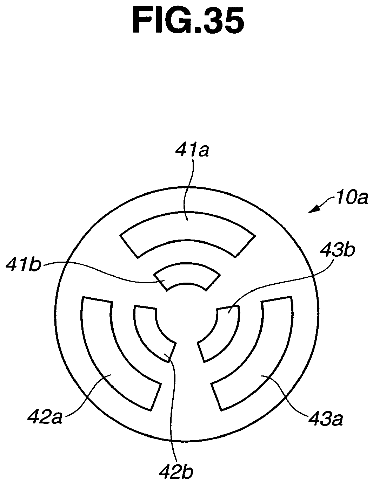

The rotary filter 10 of the present embodiment is not limited to the configuration illustrated in Fig. 13, but may also be configured, for example, as a rotary filter 10a shown in Fig. 35.

The rotary filter 10a has a first filter group made up of an optical filter 41 a that allows to pass light having a wavelength band of 600 to 650 nm, an optical filter 42a that allows to pass light having a wavelength band of 540 to 560 nm and an optical filter 43a that allows to pass light having a wavelength band of 700 to 760 nm along a circumferential direction on the outer circumference side of the disk. Furthermore, the rotary filter 10a also has a second filter group made up of an optical filter 41b that allows to pass light having a wavelength band of 600 to 650 nm, an optical filter 42b that allows to pass light having a wavelength band of 500 to 600 nm and an optical filter 43b that allows to pass light having a wavelength band of 400 to 500 nm along a circumferential direction on the inner circumference side of the disk.

According to the aforementioned configuration, upon detecting that the aforementioned first, the second or the third observation mode is selected by the mode changeover switch 15, the CPU 33 controls a filter moving mechanism (not shown) that can move the rotary filter 10a in a direction perpendicular to the optical path of the lamp 7 and thereby sets the arrangement state of the rotary filter 10a to an arrangement state in which each filter of the aforementioned first filter group can sequentially traverse the optical path of the lamp 7. Furthermore, according to the aforementioned configuration, upon detecting that aforementioned fourth observation mode is selected by the mode changeover switch 15, the CPU 33 controls the aforementioned filter moving mechanism and thereby sets the arrangement state of the rotary filter 10a to an arrangement state in which each filter of the aforementioned second filter group can sequentially traverse the optical path of the lamp 7.

Even when the light source apparatus 1 is configured to have the rotary filter 10a instead of the rotary filter 10, by matching, for example, the period during which the optical filter 41 a is inserted in the optical path of the lamp 7 to that of the optical filter 41, matching the period during which the optical filter 42a is inserted in the optical path of the lamp 7 to that of the optical filter 42 and matching the period during which the optical filter 43a is inserted in the optical path of the lamp 7 to that of the optical filter 43, it is possible to apply control on the image pickup actuator 39

(filter changeover apparatuses 39a and 39b) in the first, the second and the third observation modes as described in Fig. 17 to Fig. 21.

As described so far, the endoscope system 301 of the present embodiment has a configuration capable of adjusting the brightness of the first image signal corresponding to the first fluorescent light and the brightness of the second image signal corresponding to the second fluorescent light respectively using the brightness of the third image signal corresponding to the reference light as a reference. Therefore, the endoscope system 301 of the present embodiment can improve the diagnosis performance when making a diagnosis by causing a plurality of fluorescence probes to act on the region to be observed.

## Claims

1. A fluorescence observation apparatus comprising:
a light source section (1) that can emit a plurality of excitation light beams for exciting a plurality of fluorescent substances and a reference light having a narrow wavelength band;
an image pickup section (2) that picks up images of a plurality of fluorescent light beams emitted by emitting the plurality of excitation light beams to the plurality of fluorescent substances and reflected light of the reference light;
an image generation section (18, 19) that generates image signals corresponding to the plurality of fluorescent light beams and the reflected light of the reference light whose images are picked up by the image pickup section;
a brightness detection section (23) that detects brightness of a plurality of image signals corresponding to the plurality of fluorescent light beams and brightness of an image signal corresponding to the reflected light of the reference light, sets an upper limit value and a lower limit value of brightness using brightness of the image signal corresponding to the reflected light of the reference light as a reference and detects whether or not the brightness of the plurality of image signals corresponding to the plurality of fluorescent light beams falls within a range between the upper limit value and the lower limit value;
a brightness adjusting section (24) that adjusts brightness of each image signal outside the range out of the plurality of image signals corresponding to the plurality of fluorescent light beams so as to fall within the range, while adjusting a color tone, based on a detection result by the brightness detection section; and
an image synthesizing section (25) that assigns each of the image signals subjected to color tone adjustment by the brightness adjusting section to a different color channel and synthesizes images.

2. The fluorescence observation apparatus according to claim 1, wherein the brightness adjusting section (24) comprises:
an adjusted value setting section that calculates an adjusted value using brightness of the image signal corresponding to the reflected light of the reference light as a reference; and
an image adjusting section that performs calculation processing using the adjusted value calculated by the adjusted value setting section, the plurality of image signals corresponding to the plurality of fluorescent light beams and the image signal corresponding to the reflected light of the reference light.

3. The fluorescence observation apparatus according to claim 1, wherein the brightness adjusting section (24) comprises:
an adjusted value setting section that calculates an adjusted value using brightness of the image signal corresponding to the reflected light of the reference light as a reference; and
an exposure time control section that controls an exposure time of an image pickup device provided in the image pickup section based on the adjusted value calculated by the adjusted value setting section.

## Patentansprüche

1. Fluoreszenzbeobachtungsgerät mit:
einem Lichtquellenbereich (1), der mehrere Anregungslichtstrahlen zum Anregen einer Mehrzahl von fluoreszierenden Substanzen und ein ein schmales Wellenlängenband aufweisendes Referenzlicht abgeben kann;
einem Bildaufnahmeteil (2), das Bilder von mehreren fluoreszierenden Lichtstrahlen, die durch das Abgeben der mehreren Anregungslichtstrahlen an die Mehrzahl von fluoreszierenden Substanzen abgegeben werden, und reflektiertes Licht des Referenzlichts, aufnimmt;
einem Bilderzeugungsteil (18, 19), das Bildsignale erzeugt, die den mehreren fluoreszierenden Lichtstrahlen und dem reflektierten Licht des Referenzlichts entsprechen, dessen Bilder von dem Bildaufnahmeteil aufgenommen werden;
einem Helligkeitserfassungsteil (23), das die Helligkeit einer Mehrzahl von den mehreren fluoreszierenden Lichtstrahlen entsprechenden Bildsignalen und die Helligkeit eines dem reflektierten Licht des Referenzlichts entsprechenden Bildsignals erfasst, einen oberen Helligkeitsgrenzwert und einen unteren Helligkeitsgrenzwert festlegt, unter Verwendung der Helligkeit des dem reflektierten Licht des Referenzlichts entsprechenden Bildsignals als Bezug, und erfasst, ob die Helligkeit der Mehrzahl von Bildsignalen, die den mehreren fluoreszierenden Lichtstrahlen entsprechen, in einem Bereich zwischen dem oberen Grenzwert und dem unteren Grenzwert liegt;
einem Helligkeitseinstellungsteil (24), das die Helligkeit jedes außerhalb des Bereichs liegenden Bildsignals der Mehrzahl von den mehreren fluoreszierenden Lichtstrahlen entsprechenden Bildsignalen so einstellt, dass es in den Bereich fällt, unter Einstellung eines Farbtons, basierend auf einem Erfassungsergebnis des Helligkeitserfassungsteils; und
einem Bild-Synthesizerteil (25), das jedes der Bildsignale, die durch das Helligkeitseinstellungsteil einer Farbtoneinstellung unterzogen werden, einem anderen Farbkanal zuordnet und Bilder synthetisiert.

2. Fluoreszenzbeobachtungsgerät nach Anspruch 1, wobei das Helligkeitseinstellungsteil (24) aufweist:
einen Einstellwert-Festlegungsbereich, der einen Einstellwert berechnet und dabei die Helligkeit des dem reflektierten Licht des Referenzlichts entsprechenden Bildsignals als Bezug verwendet; und
einen Bildeinstellbereich, der eine Berechnungsverarbeitung ausführt und dabei den von dem Einstellwert-Festlegungsbereich berechneten Einstellwert verwendet, wobei die Mehrzahl von Bildsignalen den mehreren fluoreszierenden Lichtstrahlen entspricht und das Bildsignal dem reflektierten Licht des Referenzlichts entspricht.

3. Fluoreszenzbeobachtungsgerät nach Anspruch 1, wobei das Helligkeitseinstellungsteil (24) aufweist:
einen Einstellwert-Festlegungsbereich, der einen Einstellwert berechnet und dabei die Helligkeit des dem reflektierten Licht des Referenzlichts entsprechenden Bildsignals als Bezug verwendet; und
einen Belichtungszeitsteuerbereich, der eine Belichtungszeit einer in dem Bildaufnahmeteil vorgesehenen Bildaufnahmevorrichtung steuert, basierend auf dem von dem Einstellwert-Festlegungsbereich berechneten Einstellwert.

## Revendications

1. Appareil d'observation de fluorescence comprenant :
une section (1) de sources de lumière qui peut émettre une pluralité de faisceaux de lumière d'excitation pour exciter une pluralité de substances fluorescentes et une lumière de référence ayant une bande étroite de longueur d'onde ;
une section (2) de capture d'images qui capture des images d'une pluralité de faisceaux de lumière fluorescente émis en émettant la pluralité de faisceaux de lumière d'excitation sur la pluralité de substances fluorescentes et de la lumière réfléchie de la lumière de référence ;
une section (18, 19) de génération d'images qui génère des signaux d'images correspondant à la pluralité de faisceaux de lumière fluorescente et à la lumière réfléchie de la lumière de référence dont les images sont capturées par la section de capture d'images ;
une section (23) de détection de luminosité qui détecte une luminosité d'une pluralité de signaux d'images correspondant à la pluralité de faisceaux de lumière fluorescente et une luminosité d'un signal d'image correspondant à la lumière réfléchie de la lumière de référence, fixe une valeur limite supérieure et une valeur limite inférieure de luminosité en utilisant une luminosité du signal d'image correspondant à la lumière réfléchie de la lumière de référence comme une référence et détecte si la luminosité de la pluralité de signaux d'images correspondant à la pluralité de faisceaux de lumière fluorescente tombe ou non à l'intérieur d'une plage entre la valeur limite supérieure et la valeur limite inférieure ;
une section (24) d'ajustement de luminosité qui ajuste une luminosité de chaque signal d'image à l'extérieur de la plage parmi la pluralité de signaux d'images correspondant à la pluralité de faisceaux de lumière fluorescente de façon à ce qu'elle tombe à l'intérieur de la plage, tout en ajustant une tonalité chromatique, sur la base d'un résultat de détection par la section de détection de luminosité ; et
une section (25) de synthèse d'images qui attribue chacun des signaux d'images soumis à un ajustement de tonalité chromatique par la section d'ajustement de luminosité à un canal de couleur différent et synthétise des images.

2. Appareil d'observation de fluorescence selon la revendication 1, dans lequel la section (24) d'ajustement de luminosité comprend :
une section de fixation de valeur ajustée qui calcule une valeur ajustée en utilisant une luminosité du signal d'image correspondant à la lumière réfléchie de la lumière de référence comme une référence ; et
une section d'ajustement d'images qui exécute un traitement de calcul en utilisant la valeur ajustée calculée par la section de fixation de valeur ajustée, la pluralité de signaux d'images correspondant à la pluralité de faisceaux de lumière fluorescente et le signal d'image correspondant à la lumière réfléchie de la lumière de référence.

3. Appareil d'observation de fluorescence selon la revendication 1, dans lequel la section (24) d'ajustement de luminosité comprend :
une section de fixation de valeur ajustée qui calcule une valeur ajustée en utilisant une luminosité du signal d'image correspondant à la lumière réfléchie de la lumière de référence comme une référence ; et
une section de commande de durée d'exposition qui commande une durée d'exposition d'un dispositif de capture d'images prévu dans la section de capture d'images sur la base de la valeur ajustée calculée par la section de fixation de valeur ajustée.
